# EUROPEAN PATENT SPECIFICATION

(11) **EP 4 135 808 B1**
(45) Date of publication and mention of the grant of the patent: **24.01.2024**
(21) Application number: 21717158.6
(22) Date of filing: 19.03.2021
(51) Int. Cl.: A61M 16/04, A61M 25/00, B29C 45/00

(54) **REINFORCED MEDICO-SURGICAL TUBES AND THEIR MANUFACTURE**
VERSTÄRKTE MEDIZINISCH-CHIRURGISCHE SCHLÄUCHE UND IHRE HERSTELLUNG
TUBES MÉDICO-CHIRURGICAUX ET LEUR FABRICATION

(30) Priority: 15.04.2020 GB 202005470
(43) Date of publication of application: 22.02.2023
(73) Proprietor: Smiths Medical International Limited, Ashford, Kent TN25 4BF (GB)
(72) Inventor: QUACKENBUSH, John Jay, Saint Paul, MN 55127 (US); BEESON, Andrew Robert, Plymouth, MN 55442 (US); FAN, Yaling, Maple Grove, MN 55311 (US)
(74) Representative: Flint, Jonathan McNeill
(86) International application number: PCT/GB2021/000035
(87) International publication number: WO 2021/209734

(56) References cited:
- WO-A1-2015/075412
- DE-A1-102008 015 050
- DE-B3-102007 011 930
- DE-C- 867 144
- GB-A- 2 552 250
- JP-A- H 067 450

## Description

This invention relates to reinforced medico-surgical tubes of the kind having a shaft of a first, plastics material reinforced along at least a part of its length by a helical member of a second, stiffer material and embedded with the first material,
Tracheal tubes are used to enable ventilation, respiration, or spontaneous breathing of a patient. Endotracheal tubes are inserted via the mouth or nose so that one end locates in the trachea and the other end locates outside the patient. Tracheostomy tubes are inserted into the trachea via a surgically formed opening in the neck. Tracheostomy tubes can be inserted by different techniques, such as the surgical cut-down procedure carried out in an operating theatre or a cricothyroidotomy procedure, which may be carried out in emergency situations.

Tracheostomy tubes are generally used for more long-term ventilation or where it is not possible to insert an airway through the mouth or nose. The patient is often conscious while breathing through a tracheostomy tube, which may be open to atmosphere or connected by tubing to some form of ventilator. The tube is secured in position by means of a flange fixed with the machine end of the tube and positioned to extend outwardly on opposite sides of the tube.

Tracheostomy tubes can be made of various materials and are usually of a bendable plastics material such as PVC, polyurethane or silicone. Silicone tubes are particularly advantageous for long-term use because they can be highly flexible, making them less traumatic and damaging to tissue contacted by the tube. The silicone material is also highly compatible with patient tissue with a very low risk of granulation. Another advantage of silicone is that it is resistant to high temperatures, which enables it to be repeatedly autoclaved and reused. The soft nature of silicone tubes, however, means that they can be easily kinked and occluded by external pressure unless measures are taken to avoid this. Often, silicone tubes are reinforced by means of a stiff helical member extending along the tube, either along substantially their entire length or along only a part of the length. Typically, the reinforcement member is a metal wire although non-metallic helical reinforcement members, such as of stiff plastics can be used, although these do not have the same degree of kink and crush resistance as metal wires.

Examples of reinforced tracheal tubes are described in, for example, GB2552250, US5906036, WO2010/089523, EP1078645, WO2015075412, WO08083286, US6148818, US5546936, US5429127, EP0950424, EP2644221, US5628787, US4737153, US2012/0118294, US4990143, US5181509, US2007083132, US8783254, US6130406, US6017335 and DE867144.

One problem with helically-reinforced medico-surgical tubes arises where the tube needs to have a small-bore lumen or other elongate member extending along its length such as to inflate a sealing cuff or for gas sampling, irrigation or suctioning and the like. The presence of the helical reinforcement within the thickness of the wall means that it is not possible to incorporate such a small-bore lumen within the wall of the tube without increasing its thickness. Any increase in wall thickness makes the tube stiffer and, for a given outside diameter, reduces the internal diameter and thereby reduces flow along the bore of the tube. Accordingly, such helically reinforced tubes usually have any longitudinal small-bore lumen provided by a small diameter tube secured in a shallow channel along the outside of the tube. This, however, means that the otherwise smooth external surface of the tube is interrupted along its length by a projecting ridge. This is a particular disadvantage where the tube is used with delicate tissue, such as in paediatric tracheostomy tubes, since the ridge can cause damage to tissue. Similar problems exist in other reinforced medico-surgical tubes.

It is an object of the present invention to provide an alternative reinforced medico-surgical tube and a method of making such a tube.

According to one aspect of the present invention there is provided a reinforced medico-surgical tube of the above-specified kind, wherein the helical member has a longitudinal path along a part at least of its length extending transversely of the turns of the helical member, wherein the tube includes an elongate member extended along a part at least of the length of the longitudinal path so that a part at least of the thickness of the elongate member is received in the longitudinal path, and characterised in that the longitudinal path is provided by an indentation in the helical member.

According to another aspect of the present invention there is provided a reinforced medico-surgical tube of the above-specified kind, wherein the helical member has a longitudinal path along a part at least of its length extending transversely of the turns of the helical member, wherein the tube includes an elongate member extended along a part at least of the length of the longitudinal path so that a part at least of the thickness of the elongate member is received in the longitudinal path, and characterised in that the longitudinal path is provided by notches in the helical member.

According to a further aspect of the present invention there is provided a reinforced medico-surgical tube of the above-specified kind, wherein the helical member has a longitudinal path along a part at least of its length extending transversely of the turns of the helical member, wherein the tube includes an elongate member extended along a part at least of the length of the longitudinal path so that a part at least of the thickness of the elongate member is received in the longitudinal path, and characterised in that the longitudinal path is provided by a gap in the helical member.

The elongate member is preferably a small-bore tube extending longitudinally along a part at least of the length of the longitudinal path so that a part at least of the thickness of the small-bore tube is received in the longitudinal path. The tube preferably has an inflatable sealing cuff towards its patient end, the patient end of the small-bore tube opening into the sealing cuff. The helical member is preferably of a metal wire, such as of titanium. The plastics material may include silicone. The tube may be a tracheostomy tube.

According to a fourth aspect of the present invention there is provided a method of making a reinforced medico-surgical tube including the steps of providing a helical reinforcing member of relatively stiff material, deforming the outside of the helical reinforcing member inwardly to form an elongate indented groove along a part at least of the length of the reinforcing member, extending an elongate member along the groove, and overmoulding the reinforcing member and elongate member with a relatively flexible plastics material.

The method may include the steps of providing an anvil extending through the helical reinforcing member, the anvil having a groove extending longitudinally on its outside along a part at least of its length, using a die to deform a part of the helical reinforcing member into the groove on the anvil so as to form the indented groove in the helical reinforcing member, and subsequently removing the anvil and die from the reinforcing member.

A reinforced tracheostomy tube and its method of manufacture according to the present invention will now be described, by way of example, with reference to the accompanying drawings in which:
- Figure 1: is a perspective view of the tracheostomy tube;
- Figure 2: is a cross-sectional transverse view of the tube of Figure 1;
- Figure 2A: is a cross-sectional transverse view of a modified tube:
- Figure 3: is a side view of the reinforcing wire at an initial stage of manufacture;
- Figure 4: illustrates a subsequent stage in the manufacturing process;
- Figure 5: is a perspective view of the reinforcing wire after forming a groove;
- Figure 6: is a perspective view of an alternative reinforcing wire;
- Figure 7: is a perspective view of a different configuration of reinforcement; and
- Figure 8: is an end view of an alternative tube.

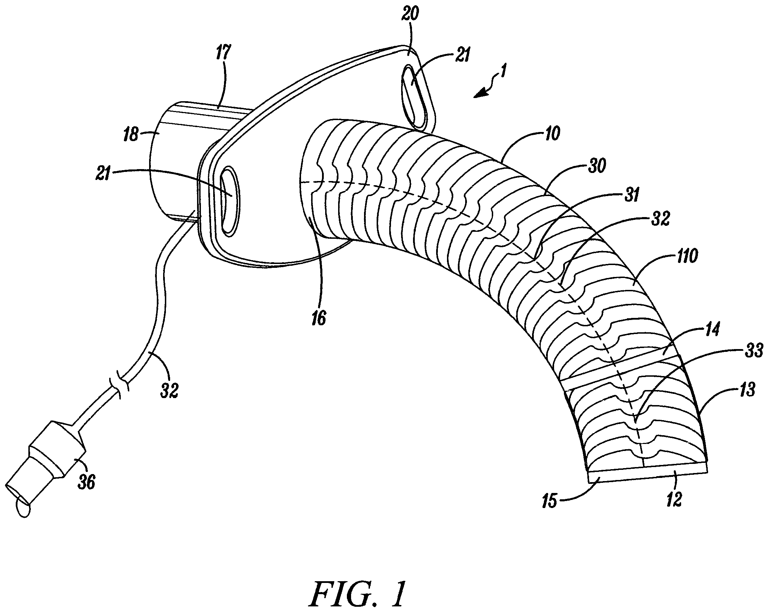

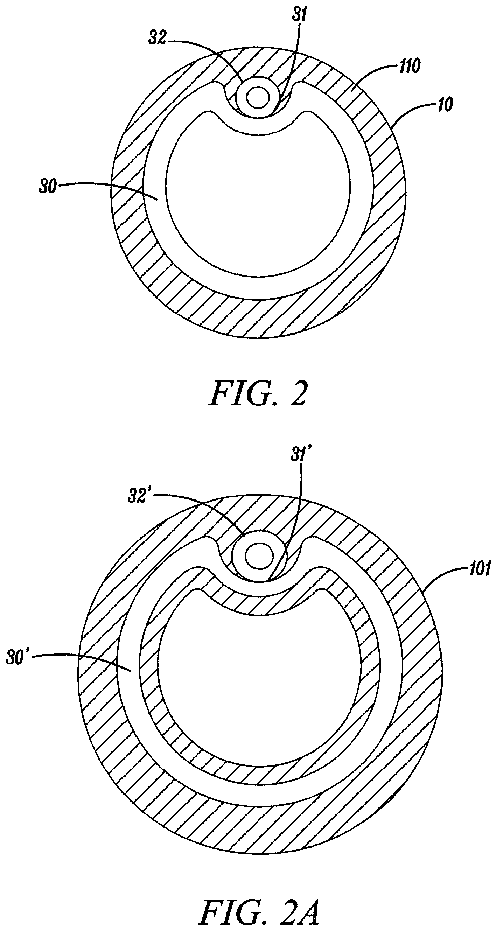

With reference first to Figures 1, 2 and 2A the tracheostomy tube 1 has a curved shaft 10 of circular section and with an outer diameter of around 12mm. The shaft 10 has a moulded, tubular wall 110 of a relatively soft plastics material. The shaft 10 has a patient end 12 adapted to be located within the trachea of the patient and has a conventional inflatable sealing cuff 13 towards its patient end. The cuff 13 shown is of the high-pressure kind made of an elastic material that lies close to the shaft 10 when deflated and is stretched outwardly when inflated. The cuff 13 is attached to the outside of the shaft 10 by two collars 14 and 15 at opposite ends. The shaft 10 and cuff 13 are both moulded of a silicone material. It will be appreciated that the tube could be of a different sizes, shapes and materials according to the application and that the cuff could be of the low-pressure kind

The machine end 16 of the shaft 10 is adapted, during use, to extend externally through the tracheostomy opening formed in the patient's neck. The machine end 16 of the shaft 10 is bonded with a hub or connector 17 having a conventional 15mm male tapered outer surface 18. The connector 17 is adapted to make a removable push fit in a conventional 15mm female connector (not shown) at one end of a breathing tube extending to a ventilator or anaesthetic machine. Alternatively, the machine end of the tube 1 could be left open to atmosphere when the patient is breathing spontaneously. The tracheostomy tube 1 also includes a radially extending support flange 20 adapted to lie against the skin surface of the neck on either side of the tracheostomy stoma. The flange 20 is moulded integrally as one part with the shaft 10 at its machine end 15, although the flange and connector could be formed separately and be subsequently attached. The flange 20 has openings 21 at opposite ends for attachment to a neck strap (not shown) used to support the tube with the patient's neck.

The shaft 10 has a reinforcement member 30 that extends along and around the shaft and is embedded with the wall 110 of the shaft by overmoulding. More particularly, the reinforcing member 30 is a helically-wound metal wire such as of titanium or stainless steel. The reinforcing wire 30 is formed with a groove 31 extending longitudinally along one side of the curve of the shaft 10, the groove being indented inwardly. A small-bore inflation line tube 32 extends along the groove 31 outside the helical reinforcement member 30 and opens at its patient end 33 beneath the trachea-sealing cuff 13 between the two collars 14 and 15. The inflation line 32 and the reinforcement member 30 are both encased in the silicone material of the wall of the shaft. The groove 31 in the reinforcement member 30 receives the thickness of the inflation line 32 sufficiently to ensure that it does not form any projection, or only forms a shallow projection, on the outside of the shaft 10. Figure 2A shows a modified tube 10' where the silicone plastics of the tube wall extends over the inner surface of the reinforcement member 30' as well as its external surface.

The machine end of the inflation line 32 extends through the flange 20 and continues unattached with the shaft 10 being terminated by a conventional combined inflation indicator and valve 36.

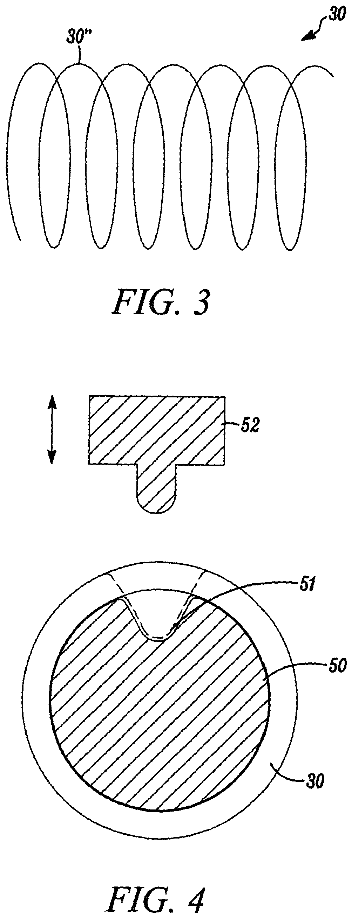

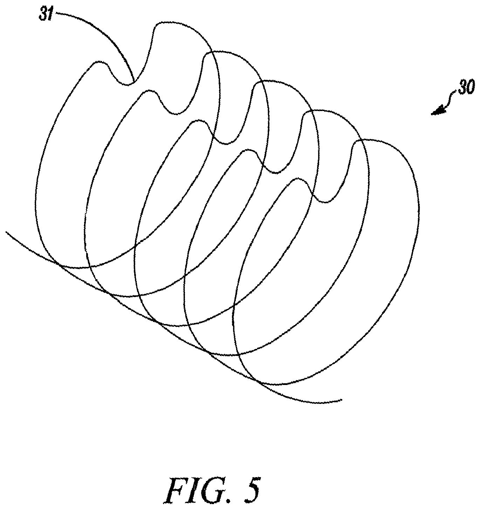

In some cases it may be preferred for the reinforcement member 30 not to extend along the entire length of the shaft, such as when it is necessary to form a region that is softer, such as at the patient end 12. In some cases, it may be necessary to form a region that is not malleable, so as to prevent the lumen being closed if the reinforced section of the tube is inadvertently compressed or pinched
The shaft 10 of the tube 1 is made using steps shown in Figures 3 to 5. First, as shown in Figure 3, a metal wire 30" is wound into a helical coil shape 30. This coil 30 may be formed initially or it may be formed by winding directly onto an anvil or former 50 shown in Figure 4. The wire 30" is shown as having a circular section but it could have other shape cross sections. For example, a wire with a rectangular or oval section could enable the wall of the tube to be thinner if the wire is wound with its wider side aligned longitudinally of the length of the tube. The anvil 50 is of cylindrical shape and circular section having a diameter equal to the desired internal diameter of the helical coil 30. The anvil 50 has a groove 51 extending along its length on its outer surface. A die 52 is positioned above the anvil 50 in alignment with the groove 51 and is mounted for reciprocating movement down into the groove and up out of the groove. With the coil 30 mounted around the anvil 50, the action of moving the die 52 downwardly causes it to engage that part of the coil above the groove 51 and deform the coil down into the groove. The metal of the coil 30 is preferably malleable so that the coil is permanently deformed with the groove 31 along its length, as shown in Figure 5. The coil 30 is then removed from the anvil 50 and placed on a suitable mould tool (not shown), the inflation line 32 being extended along the groove 31. The silicone or other soft plastics material of the wall of the shaft 10 is then overmoulded about the coil 30, flowing between its turns and covering the inflation line 32 to form a smooth outer surface of the shaft or both a smooth outer and inner surface. The flange 20 can also be integrally moulded with the shaft 10 at this stage. The sealing cuff 13 is then attached in the usual way, after forming the opening 33 to the patient end 33 of the inflation lumen 32. The machine end of the inflation line 32 is joined with the combined inflation indicator, valve and connector 36.

The arrangement of the present invention enables a tube of a relatively soft and flexible plastics material to be reinforced effectively against kinking and crushing without the inflation line or other elongate member protruding along the outer surface or protruding into the lumen of the tube.

The invention is not limited to cuffed tubes but could be used with tubes having, for example, a suction or irrigation lumen, or a lumen for gas-sampling or for supplying a therapeutic gas or liquid. Other elongate members could extend along the groove in the helical reinforcement instead of, or in addition to, a small-bore tube. The elongate member could, for example, be an electrical wire, such as for connection to an electrical sensor, or a fibre-optic cable, such as for viewing purposes or for supplying radiation to a body cavity. The invention is not limited to tracheal tubes but could be used in other medico-surgical tubes, such as urinary catheters or chest drainage tubes.

The helical reinforcement member need not be of a metal but could, for example, be of a stiff plastics material. With some materials it might be necessary to use a heated die to enable the coil to be deformed and to set in the groove shape. The reinforcement member need not extend along the entire length of the tube since in some tubes it might only be necessary to reinforce a part of the tube. In some tubes the small bore tube or other elongate member might only extend along a part of the length of the reinforced portion of the tube, in which case the groove in the outside of the coil need not extend along its entire length.

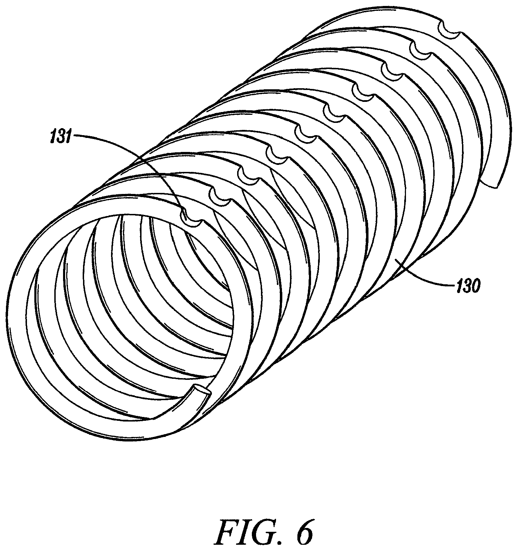

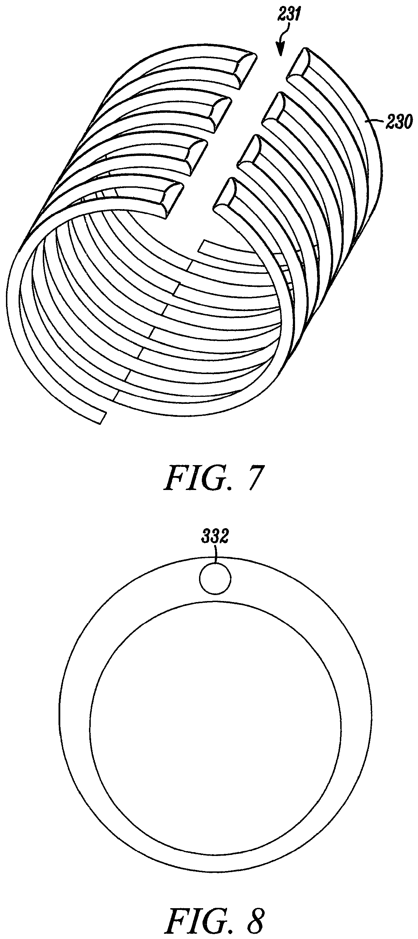

Instead of an indented notch in the reinforcement member, as shown in Figures 1, 2, 4 and 5, a longitudinal path could be provided along the reinforcement member in other ways. For example, a longitudinal path 130 could be provided by a cut-out notch 131, as shown in Figure 6. A path for receiving an elongate member could be provided in other ways, such as shown in Figure 7 where the turns of the reinforcement coil 230 are bent back on themselves in boustrophedon fashion to leave a path 231 extending longitudinally along the coil. The bent portions of the coil 230 would preferably have rounded corners and not the square corners shown in the Figure. Figure 8 shows how the wall thickness could vary around the circumference of the tube, being thickest in the region where the elongate member 332 extends and without any protrusion on the inner or outer surface.

## Claims

1. A reinforced medico-surgical tube (1) having a shaft (10, 10') of a first, plastics material reinforced along at least a part of its length by a helical member (30, 30', 130, 230) of a second, stiffer material and embedded with the first material, wherein the helical member (30, 30', 130, 230) has a longitudinal path (31, 31', 131, 231) along a part at least of its length extending transversely of the turns of the helical member (30, 30', 130, 230), wherein the tube (1) includes an elongate member (32, 32') extended along a part at least of the length of the longitudinal path (31, 31', 131, 231) so that a part at least of the thickness of the elongate member (32, 32') is received in the longitudinal path (31, 31', 131, 231), and **characterised in that** the longitudinal path is provided by an indentation (31, 31') in the helical member (30, 30').

2. A reinforced medico-surgical tube (1) having a shaft (10, 10') of a first, plastics material reinforced along at least a part of its length by a helical member (30, 30', 130, 230) of a second, stiffer material and embedded with the first material, wherein the helical member (30, 30', 130, 230) has a longitudinal path (31, 31', 131, 231) along a part at least of its length extending transversely of the turns of the helical member (30, 30', 130, 230), wherein the tube (1) includes an elongate member (32, 32') extended along a part at least of the length of the longitudinal path (31, 31', 131, 231) so that a part at least of the thickness of the elongate member (32, 32') is received in the longitudinal path (31, 31', 131, 231), and **characterised in that** the longitudinal path is provided by notches (131) in the helical member (130).

3. A reinforced medico-surgical tube (1) having a shaft (10, 10') of a first, plastics material reinforced along at least a part of its length by a helical member (30, 30', 130, 230) of a second, stiffer material and embedded with the first material, wherein the helical member (30, 30', 130, 230) has a longitudinal path (31, 31', 131, 231) along a part at least of its length extending transversely of the turns of the helical member (30, 30', 130, 230), wherein the tube (1) includes an elongate member (32, 32') extended along a part at least of the length of the longitudinal path (31, 31', 131, 231) so that a part at least of the thickness of the elongate member (32, 32') is received in the longitudinal path (31, 31', 131, 231), and **characterised in that** the longitudinal path is provided by a gap (231) in the helical member (230).

4. A tube according to any one of the preceding claims, **characterised in that** the elongate member is a small-bore tube (32) extending longitudinally along a part at least of the length of the longitudinal path (31, 31', 131, 231) so that a part at least of the thickness of the small-bore tube (32) is received in the longitudinal path (31, 31', 131, 231).

5. A tube according to Claim 4, **characterised in that** the tube has an inflatable sealing cuff (13) towards its patient end (12), and that the patient end (33) of the small-bore tube (32) opens into the sealing cuff (13).

6. A tube according to any one of the preceding claims, **characterised in that** the helical member (30, 30', 130, 230) is of a metal wire.

7. A tube according to Claim 6, **characterised in that** the metal wire is of titanium.

8. A tube according to any one of the preceding claims, **characterised in that** the plastics material includes silicone.

9. A tube according to any one of the preceding claims wherein the tube is a tracheostomy tube (1).

10. A method of making a reinforced medico-surgical tube including the steps of providing a helical reinforcing member (30, 30') of relatively stiff material, deforming the outside of the helical reinforcing member inwardly to form an elongate indented groove (31, 31') along a part at least of the length of the reinforcing member, extending an elongate member (32) along the groove (31, 31'), and overmoulding the reinforcing member (30, 30') and elongate member (32) with a relatively flexible plastics material (110).

11. A method according to Claim 10, **characterised in that** the method includes the steps of providing an anvil (50) extending through the helical reinforcing member (30, 30'), the anvil (50) having a groove (51) extending longitudinally on its outside along a part at least of its length, using a die (52) to deform a part of the helical reinforcing member (30, 30') into the groove (51) on the anvil (50) so as to form the indented groove (31, 31') in the helical reinforcing member, and subsequently removing the anvil and die from the reinforcing member.

## Patentansprüche

1. Verstärkter medizinisch-chirurgischer Tubus (1) mit einem Schaft (10, 10') aus einem ersten Kunststoffmaterial, der entlang mindestens eines Teils seiner Länge durch ein in das erste Material eingebettetes wendeiförmiges Element (30, 30', 130, 230) aus einem zweiten, steiferen Material verstärkt ist, wobei das wendelförmige Element (30, 30', 130, 230) eine Längsbahn (31, 31', 131, 231) entlang mindestens eines Teils seiner Länge aufweist, der sich quer zu den Windungen des wendelförmigen Elements (30, 30', 130, 230) erstreckt, wobei der Tubus (1) ein längliches Element (32, 32') aufweist, das sich entlang eines Teils zumindest der Länge der Längsbahn (31, 31', 131, 231) erstreckt, so dass ein Teil zumindest der Dicke des länglichen Elements (32, 32') in der Längsbahn (31, 31', 131, 231) aufgenommen wird, **dadurch gekennzeichnet, dass** die Längsbahn durch eine Vertiefung (31, 3') in dem wendelförmigen Element (30, 30') vorgesehen ist.

2. Verstärkter medizinisch-chirurgischer Tubus (1) mit einem Schaft (10, 10') aus einem ersten Kunststoffmaterial, der entlang mindestens eines Teils seiner Länge durch ein in das erste Material eingebettetes wendelförmiges Element (30, 30', 130, 230) aus einem zweiten, steiferen Material verstärkt ist, wobei das wendelförmige Element (30, 30', 130, 230) eine Längsbahn (31, 31', 131, 231) entlang mindestens eines Teils seiner Länge aufweist, der sich quer zu den Windungen des wendelförmigen Elements (30, 30', 130, 230) erstreckt, wobei der Tubus (1) ein längliches Element (32, 32') aufweist, das sich entlang eines Teils zumindest der Länge der Längsbahn (31, 31', 131, 231) erstreckt, so dass ein Teil zumindest der Dicke des länglichen Elements (32, 32') in der Längsbahn (31, 31', 131, 231) aufgenommen wird, **dadurch gekennzeichnet, dass** der Längsweg durch Kerben (131) in dem wendelförmigen Element (130) bereitgestellt wird.

3. Verstärkter medizinisch-chirurgischer Tubus (1) mit einem Schaft (10, 10') aus einem ersten Kunststoffmaterial, der entlang mindestens eines Teils seiner Länge durch ein in das erste Material eingebettetes wendelförmiges Element (30, 30', 130, 230) aus einem zweiten, steiferen Material verstärkt ist, wobei das wendelförmige Element (30, 30', 130, 230) eine Längsbahn (31, 31', 131, 231) entlang mindestens eines Teils seiner Länge aufweist, der sich quer zu den Windungen des wendelförmigen Elements (30, 30', 130, 230) erstreckt, wobei der Tubus (1) ein längliches Element (32, 32') aufweist, das sich entlang eines Teils zumindest der Länge der Längsbahn (31, 31', 131, 231) erstreckt, so dass ein Teil zumindest der Dicke des länglichen Elements (32, 32') in der Längsbahn (31, 31', 131, 231) aufgenommen wird, **dadurch gekennzeichnet, dass** der Längsweg durch einen Spalt (231) in dem wendelförmigen Element (230) bereitgestellt wird.

4. Tubus nach einem der vorhergehenden Ansprüche, **dadurch gekennzeichnet, dass** das längliche Element ein kleinlumiger Tubus (32) ist, das sich in Längsrichtung über mindestens einen Teil der Länge der Längsbahn (31, 31', 131, 231) erstreckt, so dass mindestens ein Teil der Dicke des kleinlumigen Tubus (32) in der Längsbahn (31, 31', 131, 231) aufgenommen wird.

5. Tubus nach Anspruch 4, **dadurch gekennzeichnet, dass** der Tubus zu seinem Patientenende (12) hin eine einschiebbare Dichtungsmanschette (13) aufweist und dass das Patientenende (33) des kleinlumigen Tubus (32) in die Dichtungsmanschette (13) mündet.

6. Tubus nach einem der vorhergehenden Ansprüche, **dadurch gekennzeichnet, dass** das spiralförmige Element (30, 30', 130, 230) aus einem Metalldraht besteht.

7. Tubus nach Anspruch 6, **dadurch gekennzeichnet, dass** der Metalldraht aus Titan besteht.

8. Tubus nach einem der vorhergehenden Ansprüche, **dadurch gekennzeichnet, dass** das Kunststoffmaterial Silikon enthält.

9. Tubus nach einem der vorhergehenden Ansprüche, wobei der Tubus ein Tracheostomie-Tubus (1) ist.

10. Verfahren zur Herstellung eines verstärkten medizinisch-chirurgischen Tubus, umfassend die Schritte des Bereitstellens eines wendeiförmigen Verstärkungselements (30, 30') aus relativ steifem Material, des Verformens der Außenseite des wendelförmigen Verstärkungselements nach innen, um eine längliche, vertiefte Nut (31, 31') entlang mindestens eines Teils der Länge des Verstärkungselements zu erhalten, des Erstreckens eines länglichen Elements (32) entlang der Nut (31, 31'), und des Überformens des Verstärkungselements (30, 30') und des länglichen Elements (32) mit einem relativ flexiblen Kunststoffmaterial (110).

11. Verfahren nach Anspruch 10, **dadurch gekennzeichnet, dass** das Verfahren die folgenden Schritte umfasst: Bereitstellen eines Ambosses (50), der sich durch das wendelförmige Verstärkungselement (30, 30') erstreckt, wobei der Amboss (50) eine Nut (51) aufweist, die sich in Längsrichtung an seiner Außenseite entlang mindestens eines Teils seiner Länge erstreckt, Verwenden einer Matrize (52), um einen Teil des wendelförmigen Verstärkungselements (30, 30') in die Nut (51) auf dem Amboss (50) zu verformen, um die eingedrückte Nut (31, 31') in dem wendeiförmigen Verstärkungselement zu bilden, und anschließendes Entfernen des Ambosses und der Matrize von dem Verstärkungselement.

## Revendications

1. Tube médico-chirurgical renforcé (1) comportant une tige (10, 10') en un premier matériau plastique renforcé sur au moins une partie de sa longueur par un élément hélicoïdal (30, 30', 130, 230) en un second matériau plus rigide et intégré au premier matériau,
dans lequel
l'élément hélicoïdal (30, 30', 130, 230) a une trajectoire longitudinale (31, 31', 131, 231) le long d'une partie au moins de sa longueur s'étendant transversalement aux spires de l'élément hélicoïdal (30, 30', 130, 230), dans lequel le tube (1) comprend un élément allongé (32, 32') s'étendant le long d'une partie au moins de la longueur de la trajectoire longitudinale (31, 31', 131, 231) de sorte qu'une partie au moins de l'épaisseur de l'élément allongé (32, 32') soit reçue dans la trajectoire longitudinale (31, 31', 131, 231), et **caractérisé en ce que** la trajectoire longitudinale est fournie par une échancrure (31, 31') dans l'élément hélicoïdal (30, 30').

2. Tube médico-chirurgical renforcé (1) comportant une tige (10, 10') en un premier matériau plastique renforcé sur au moins une partie de sa longueur par un élément hélicoïdal (30, 30', 130, 230) en un second matériau plus rigide et intégré au premier matériau,
dans lequel
l'élément hélicoïdal (30, 30', 130, 230) a une trajectoire longitudinale (31, 31', 131, 231) le long d'une partie au moins de sa longueur s'étendant transversalement aux spires de l'élément hélicoïdal (30, 30', 130, 230), dans lequel le tube (1) comprend un élément allongé (32, 32') s'étendant le long d'une partie au moins de la longueur de la trajectoire longitudinale (31, 31', 131, 231) de sorte qu'une partie au moins de l'épaisseur de l'élément allongé (32, 32') soit reçue dans la trajectoire longitudinale (31, 31', 131, 231), et **caractérisé en ce que** la trajectoire longitudinale est fournie par des encoches (131) dans l'élément hélicoïdal (130).

3. Tube médico-chirurgical renforcé (1) comportant une tige (10, 10') en un premier matériau plastique renforcé sur au moins une partie de sa longueur par un élément hélicoïdal (30, 30', 130, 230) en un second matériau plus rigide et intégré au premier matériau,
dans lequel
l'élément hélicoïdal (30, 30', 130, 230) a une trajectoire longitudinale (31, 31', 131, 231) le long d'une partie au moins de sa longueur s'étendant transversalement aux spires de l'élément hélicoïdal (30, 30', 130, 230), dans lequel le tube (1) comprend un élément allongé (32, 32') s'étendant le long d'une partie au moins de la longueur de la trajectoire longitudinale (31, 31', 131, 231) de sorte qu'une partie au moins de l'épaisseur de l'élément allongé (32, 32') soit reçue dans la trajectoire longitudinale (31, 31', 131, 231), et **caractérisé en ce que** la trajectoire longitudinale est fournie par un espace (231) dans l'élément hélicoïdal (230).

4. Tube selon l'une quelconque des revendications précédentes, **caractérisé en ce que** l'élément allongé est un tube de petit diamètre (32) s'étendant longitudinalement le long d'une partie au moins de la longueur de la trajectoire longitudinale (31, 31', 131, 231) de sorte qu'une partie au moins de l'épaisseur du tube de petit calibre (32) est reçue dans la trajectoire longitudinale (31, 31', 131, 231).

5. Tube selon la revendication 4, **caractérisé en ce que** le tube présente une manchette d'étanchéité gonflable (13) vers son extrémité patient (12), et que l'extrémité patient (33) du tube de petit calibre (32) débouche dans la manchette d'étanchéité (13).

6. Tube selon l'une quelconque des revendications précédentes, **caractérisé en ce que** l'élément hélicoïdal (30, 30', 130, 230) est en fil métallique.

7. Tube selon la revendication 6, **caractérisé en ce que** le fil métallique est en titane.

8. Tube selon l'une quelconque des revendications précédentes, **caractérisé en ce que** le matériau plastique comprend du silicone.

9. Tube selon l'une quelconque des revendications précédentes, dans lequel le tube est un tube de trachéotomie (1).

10. Procédé de fabrication d'un tube médico-chirurgical renforcé comprenant les étapes consistant à
fournir un élément de renforcement hélicoïdal (30, 30') en matériau relativement rigide, déformer l'extérieur de l'élément de renforcement hélicoïdal vers l'intérieur pour former une rainure allongée échancrée (31, 31') le long d'une partie au moins de la longueur de l'élément de renforcement, étendre un élément allongé (32) le long de la rainure (31, 31'), et surmouler l'élément de renforcement (30, 30') et l'élément allongé (32) avec un matériau plastique relativement souple (110).

11. Procédé selon la revendication 10, **caractérisé en ce que** le procédé comprend les étapes consistant à
fournir une enclume (50) s'étendant à travers l'élément de renforcement hélicoïdal (30, 30'), l'enclume (50) ayant une rainure (51) s'étendant longitudinalement sur sa face extérieure sur une partie au moins de sa longueur, utiliser une matrice (52) pour déformer une partie de l'élément de renforcement hélicoïdal (30, 30') dans la rainure (51) sur l'enclume (50) de manière à former la rainure échancrée (31, 31') dans l'élément de renforcement hélicoïdal, et ensuite retirer l'enclume et la matrice de l'élément de renforcement.
